# EUROPEAN PATENT SPECIFICATION

(11) **EP 0 366 328 B1**
(45) Date of publication and mention of the grant of the patent: **19.05.1993**
(21) Application number: 89310624.5
(22) Date of filing: 17.10.1989
(51) Int. Cl.: C07D 231/12, C07D 401/04

(54) **Process for preparing 4-methylpyrazoles**
Verfahren zur Herstellung von 4-Methylpyrazolen
Procédé pour la préparation des 4-méthylpyrazoles

(30) Priority: 26.10.1988 JP 270345/88; 12.09.1989 JP 236173/89
(43) Date of publication of application: 02.05.1990
(73) Proprietor: NISSAN CHEMICAL INDUSTRIES, LIMITED, Chiyoda-ku Tokyo 101 (JP)
(72) Inventor: Murakami, Hiroshi c/o Central Research Inst., Funabashi-shi Chiba-ken (JP); Yamamoto, Susumu c/o Central Research Inst., Funabashi-shi Chiba-ken (JP); Iwasawa, Yoshihiro c/o Central Research Inst., Funabashi-shi Chiba-ken (JP); Suzuki, Kenji c/o Central Research Inst., Funabashi-shi Chiba-ken (JP); Suzuki, Fumio c/o Central Research Inst., Funabashi-shi Chiba-ken (JP); Hashiba, Isao c/o Onoda Factory, NISSAN CHEMICAL, Onoda-shi Yamaguchi-ken (JP)
(74) Representative: Brock, Peter William

(56) References cited:
- EP-A- 0 020 964
- DE-A- 3 122 261
- GB-A- 1 368 615
- CHEMICAL ABSTRACTS, vol. 55, no. 19, September 18, 1961, Columbus, Ohio, US; SCOTT SEARLES et al.; & Journal of Organic Chemistry, vol. 20, 1960, pages 36-40
- CHEMICAL ABSTRACTS, vol. 80, no. 21, May 27, 1974, page 352, abstra ct no. 119819r, Columbus, Ohio, US; BOULET PHILIPPE et al.; & Bull. Soc. Chim. Fr. 1973 (11, Pt. 2) pages 3159-3162

## Description

This invention relates to a process for preparing 4-methylpyrazoles useful as intermediates for pharmaceuticals and agricultural chemicals.

In the prior art, various processes for preparing pyrazoles have been known as described below.
(1) Chemisch Berichte, vol. 59, p. 610 discloses a process for preparing 1,4-dimethylpyrazole by decarboxylation of 1,4-dimethyl-5-pyrazole carboxylic acid.
   However, this process cannot be said to be an industrial production process, because synthesis of 1,4-dimethyl-5-pyrazole carboxylic acid is complicated, and moreover the synthesis steps are lengthy.
(2) Japanese Provisional Patent Publication No. 106620/1982 discloses a reaction between dibromo-2-methylpropanal and hydrazine and 4-methylpyrazole can be obtained at a yield of 22 %.
   This method uses expensive 2,3-dibromo-2-methylpropanal and also the yield is low.
(3) DE-A-3122261 discloses the reaction between 2,3-dichloropropanal and hydrazines in the presence of an equivalent amount or more of sodium hydroxide usually under strong alkaline condition, and, for example, pyrazole is obtained at a yield of 75 %, 1-methylpyrazole at a yield of 49 % and 1-phenylpyrazole at a yield of 54 %.

However, this process concerns a process for preparing pyrazoles having no substituent at the 4-position, and when this process was applied for preparation of 4-methylpyrazoles, no good result can be obtained (see Comparative example 1 shown hereinbelow).

There has been known that 2,3-dichloropropanal becomes 2-chloroacrolein at high temperature or in the presence of a base by rapid dehydrochlorination [Chemical Abstract, vol. 52, 1208i, vol. 83, 42773y]. According to the present inventors' investigation, it can be found that the above reaction occurs similarly in the presence of water under a strong alkaline condition (Reaction scheme I).

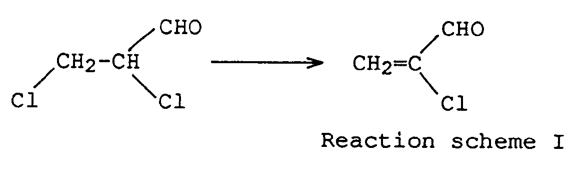

Formed 2-chloroacrolein can produce pyrazole ring by reacting with hydrazines.

On the other hand, in the case of 2,3-dichloro-2-methylpropanal which is a starting material in the present invention, it is easily decomposed in the presence of water under a strong alkaline condition and no formation of 3-chloro-2-methylpropenal can be admitted (found according to the present inventors' investigation).

Generally, as has been known that in aldehyde having no hydrogen atom at α-position, Cannizzaro reaction takes priority in a strong alkaline aqueous solution. In the case of 2,3-dichloro-2-methylpropanal of the present invention, it can easily be estimated that the same reaction will occur.

As described above, since reactivity and stability of aldehyde 2,3-dichloro-2-methylpropanal having no hydrogen atom at α-position are quite different from aldehyde 2,3-dichloropropanal having hydrogen atom at α-position, it would be impossible to apply the reaction conditions disclosed in the above (3) to a preparation of 4-methylpyrazoles as it were in the reaction with hydrazines.

The present inventors have found that decomposition of 2,3-dichloro-2-methylpropanal can possibly be depressed by controling pH in the reaction system in the reaction of 2,3-dichloro-2-methylpropanal and hydrazines, and further investigated intensively about reaction conditions therefor, and consequently accomplished a process for preparing 4-methylpyrazoles industrially advantageously, that is, the present invention.

More specifically, the present invention concerns a process for preparing 4-methylpyrazoles represented by the formula (II): 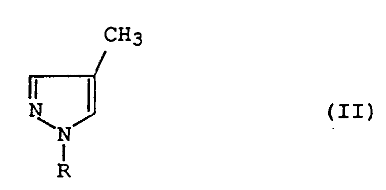
(wherein R represents a hydrogen atom, an alkyl group having 1 to 4 carbon atoms, a substituted or unsubstituted phenyl group (as defined below) or a substituted or unsubstituted pyridyl group (as defined below), which comprises reacting 2,3-dichloro-2-methyl-propanal with hydrazines represented by the formula (I):

RNHNH₂ (I)

(wherein R is the same as defined above).

According to the invention, the reaction is carried out at a temperature of -10 to 160°C, in the presence or absence of a solvent, with the pH being controlled at a value of 11 or lower when reaction is carried out in water or a solvent containing water, or in a two layer system using water and a water-insoluble organic solvent.

In the above formula (II), the alkyl group of R having 1 to 4 carbon atoms may include a methyl group, an ethyl group, a n-propyl group, an i-propyl group, a n-butyl group, an i-butyl group or a t-butyl group.

The substituted or unsubstituted phenyl group is a phenyl group, a 2-methylphenyl group, a 3-methylphenyl group, a 4-methylphenyl group, a 2-ethylphenyl group, a 3-ethylphenyl group, a 4-ethylphenyl group, a 2-n-propylphenyl group, a 2-i-propylphenyl group, a 3-n-propylphenyl group, a 3-i-propylphenyl group, a 4-n-propylphenyl group, a 4-i-propylphenyl group, a 2-t-butylphenyl group, a 4-n-butylphenyl group, a 4-i-butylphenyl group, a 4-t-butylphenyl group, a 2,4-dimethylphenyl group, a 2,6-dimethylphenyl group, a 2,4-diethylphenyl group, a 2,6-diethylphenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-n-propoxyphenyl group, a 2-i-propoxyphenyl group, a 3-n-propoxyphenyl group, a 4-n-propoxyphenyl group, a 4-i-propoxyphenyl group, a 2-n-butoxyphenyl group, a 4-n-butoxyphenyl group, a 4-i-butoxyphenyl group, a 4-t-butoxyphenyl group, a 2-chlorophenyl group, a 3-chlorophenyl group, a 4-chlorophenyl group, a 2-fluorophenyl group, a 3-fluorophenyl group, a 4-fluorophenyl group, a 2,4-dichlorophenyl group, a 2,4-difluorophenyl group, a 2,6-dichlorophenyl group, a 2,6-difluorophenyl group, a 2-nitrophenyl group, a 3-nitrophenyl group, a 4-nitrophenyl group, a 2-difluoromethoxyphenyl group, a 3-difluoromethoxyphenyl group or a 4-difluoromethoxyphenyl group. The substituted or unsubstituted pyridyl group is a 2-pyridyl group, a 3-methyl-2-pyridyl group, a 4-methyl-2-pyridyl group, a 5-methyl-2-pyridyl group, a 6-methyl-2-pyridyl group, a 3-chloro-2-pyridyl group, a 4-chloro-2-pyridyl group, a 5-chloro-2-pyridyl group, a 6-chloro-2-pyridyl group, a 3,5-dichloro-2-pyridyl group, a 3-trifluoromethyl-2-pyridyl group, a 5-trifluoromethyl-2-pyridyl group, a 3-pyridyl group, a 2-methyl-3-pyridyl group, a 4-methyl-3-pyridyl group, a 5-methyl-3-pyridyl group, a 6-methyl-3-pyridyl group, a 2-chloro-3-pyridyl group, a 5-chloro-3-pyridyl group, a 6-chloro-3-pyridyl group, a 4-pyridyl group, a 2-methyl-4-pyridyl group, a 3-methyl-4-pyridyl group, a 2-chloro-4-pyridyl group or a 3-chloro-4-pyridyl group. In the above, n means normal, i means iso and t means tertiary, respectively.

Specific examples of the 4-methylpyrazoles of the formula (II) may include 4-methylpyrazole, 1,4-dimethylpyrazole, 1-ethyl-4-methylpyrazole, 1-n-propyl-4-methylpyrazole, 1-i-propyl-4-methylpyrazole, 1-n-butyl-4-methylpyrazole, 1-i-butyl-4-methylpyrazole, 1-t-butyl-4-methylpyrazole, 1-phenyl-4-methylpyrazole, 1-(2-pyridyl)-4-methylpyrazole, 1-(3-pyridyl)-4-methylpyrazole, or 1-(4-pyridyl)-4-methylpyrazole. In the above, n means normal, i means iso and t means tertiary, respectively.

2,3-Dichloro-2-methylpropanal can be obtained by the known reaction between methacrolein and chlorine [Journal of Organic Chemistry, vol. 26, p. 36 (1961); Chemical Abstract vol. 83, 42773y].

Specific examples of hydrazines of the formula (I) may include hydrazine, methylhydrazine, ethylhydrazine, n-propylhydrazine, i-propylhydrazine, n-butylhydrazine, i-butylhydrazine, t-butylhydrazine, phenylhydrazine, 2-methylphenylhydrazine, 3-methylphenylhydrazine, 4-methylphenylhydrazine, 2,6-dimethylphenylhydrazine, 2-ethylphenylhydrazine, 2-methoxyphenylhydrazine, 3-methoxyphenylhydrazine, 4-methoxyphenylhydrazine, 2-chlorophenylhydrazine, 3-chlorophenylhydrazine, 4-chlorophenylhydrazine, 4-fluorophenylhydrazine, 2,4-dichlorophenylhydrazine, 2-nitrophenylhydrazine, 3-nitrophenylhydrazine, 4-nitrophenylhydrazine, 2-pyridylhydrazine, 3-methyl-2-pyridylhydrazine, 6-methyl-2-pyridylhydrazine, 3-chloro-2-pyridylhydrazine, 6-chloro-2-pyridylhydrazine, 3-pyridylhydrazine, and 4-pyridylhydrazine. In the above, n means normal, i means iso and t means tertiary, respectively.

The above hydrazines can be also formed as the salts such as hydrochlorides, sulfates, nitrates, and acetates.

The hydrazines of the formula (I) are not required to be of high purity, but can be also used under the state of commercially available aqueous solutions.

The amount of the hydrazines of the formula (I) used may be generally in the range of 0.7 to 3.0 moles of the hydrazines of the formula (I), preferably 1.0 to 2.0 moles, based on 1 mole of 2,3-dichloro-2-methylpropanal.

As the reaction temperature, a range of -10 to 160 °C is employed.

Particularly, when R is a hydrogen atom, a range of 20 to 150 °C is preferred, and when R is an alkyl group having 1 to 4 carbon atoms or a substituted or unsubstituted phenyl group or a substituted or unsubstituted pyridyl group, a range of 0 to 90 °C is preferred.

The present invention can be practiced with no solvent, but a solvent can be also used.

As the solvent, there may be included aliphatic hydrocarbons such as hexane, or heptane; halogenated aliphatic hydrocarbons such as methylene chloride, dichloroethane, chloroform, carbon tetrachloride, or tetrachloroethane; aromatic hydrocarbons such as benzene, toluene, or xylene; halo-substituted aromatic hydrocarbons such as chlorobenzene, or dichlorobenzene; alcohols such as methanol, or ethanol; ethers such as diethyl ether, tetrahydrofuran, or dioxane; lower fatty acids such as acetic acid, propionic acid, or butyric acid; water and aqueous solvents such as hydrochloric acid, or sulfuric acid.

The concentration of the aqueous hydrochloric acid solution may be generally 0.01 to 50 % by weight, and the concentration of the aqueous sulfuric acid solution may be generally 0.01 to 80 % by weight.

The above solvents can be also used as a mixture of two or more kinds.

As the solvent, one or two or more solvents selected from water, aqueous hydrochloric acid solution, halogenated aliphatic hydrocarbon solvents such as dichloroethane, ether solvents such as diethyl ether, halo-substituted aromatic hydrocarbons such as o-dichlorobenzene, alcohols such as methanol, and lower fatty acids such as acetic acid or butyric acid, are particularly preferred.

The amount of the solvent used may be generally in the range of 0.1 to 10 parts by weight, preferably 0.3 to 5 parts by weight, based on 1 part by weight of 2,3-dichloro-2-methylpropanal.

The reaction of the present invention may be possible in the absence of a dehydrochlorinating agent, but the dehydrochlorinating agent may be added depending on necessity.

As the dehydrochlorinating agent, inorganic or organic bases which can usually be employed may be used. For example, there may be mentioned aliphatic amines such as trimethylamine, triethylamine, tri-n-propylamine, tributylamine, tri-n-octylamine, or diisopropylethylamine, dimethylcyclohexylamine; aromatic amines such as N,N-dimethylaniline, or N,N-diethylaniline; pyridines such as pyridine, picoline, or ethylmethylpyridine; or inorganic salts such as sodium hydroxide, potassium hydroxide, sodium carbonate, potassium carbonate, sodium hydrogen carbonate, or potassium hydrogen carbonate. Also, it may be also possible to excessively use hydrazines which are starting materials in the present invention as the dehydrochlorinating agent. The dehydrochlorinating agent may be used in combination of two or more.

A ratio of 2,3-dichloro-2-methylpropanal, hydrazines and dehydrochlorinating agent is that 0.7 to 3 moles of hydrazines and 0 to 3 moles of the dehydrochlorinating agent are generally used based on 1 mole of 2,3-dichloro-2-methylpropanal. If necessary, 1 to 2 moles of hydrazines and 1 to 2 moles of the dehydrochlorinating agent may be used.

The reaction of the present invention proceeds under the acidic or alkaline conditions of pH being 11 or less when the reaction is carried out in a hydrated system and a yield is further heightened by adjusting the pH in the reaction system to 4 to 9.

However, in the case of strong alkaline condition in the presence of water, decomposition of 2,3-dichloro-2-methylpropanal which is the starting material occurs as described above, so that a yield of the compound of the present invention represented by the formula (II) is remarkably lowered (see Comparative example 1 hereinbelow).

The reaction of the present invention may be possible either homogeneous system or two layer system, but a yield is further increased by using a catalytic amount of an organic base and simultaneously adjusting a pH of the reaction system to 11 or less, preferably 4 to 9 by addition of an inorganic base. The organic base as a catalyst may include aliphatic amines such as trimethylamine, triethylamine, tri-n-propylamine, tributylamine, tri-n-octylamine, diisopropylethylamine, dimethylcyclohexylamine or diethylcyclohexylamine; aromatic amines such as N,N-dimethylaniline, or N,N-diethylaniline; and pyridines such as pyridine, picoline, or ethylmethylpyridine. The inorganic base may include sodium hydroxide, potassium hydroxide, sodium carbonate, or potassium carbonate.

Further, in the case of two layer system, a yield is also increased by using a phase-transfer catalyst, and simultaneously adjusting the pH of the reaction system to 11 or less, preferably 4 to 9, by addition of an inorganic base. The phase-transfer catalyst may include tetrabutylammonium bromide, triethylbenzylammonium chloride, tetrabutylammonium hydrosulfate, tetraoctylmethylamminium chloride, cetyltrimethylammonium bromide, tetrabutylphosphonium chloride, tetraphenylphosphonium chloride, 18-crown-6, or PEG-1000.

As the reaction method between 2,3-dichloro-2-methylpropanal and hydrazines of the formula (I), there may be employed (1) the method in which the reaction is carried out by adding dropwise 2,3-dichloro-2-methylpropanal into hydrazines, (2) the method in which 2,3-dichloro-2-methylpropanal is added dropwise into a mixture of hydrazines and a dehydrochlorinating agent, (3) the method in which 2,3-dichloro-2-methylpropanal and a dehydrochlorinating agent are simultaneously added dropwise into hydrazines, (4) the method in which hydrazines are added dropwise into 2,3-dichloro-2-methylpropanal, (5) the method in which hydrazines and a dehydrochlorinating agent are simultaneously added dropwise into 2,3-dichloro-2-methylpropanal, or (6) the method in which hydrazines and 2,3-dichloro-2-methylpropanal are simultaneously added dropwise into a solvent.

In the above operating methods, 2,3-dichloro-2-methylpropanal and the hydrazines may be each in either the state not diluted, or the state of a solution or a dispersion for carrying out the reaction.

As the post-treatment operations after completion of the reaction, ordinarily an alkali such as sodium carbonate, potassium carbonate, sodium hydroxide, potassium hydroxide is added into the reaction mixture to neutralize the reaction mixture, and if necessary, the reaction product is extracted with an organic solvent, followed by distillation, to give 4-methylpyrazoles of the formula (II).

The 4-methylpyrazoles of the formula (II) wherein R is an alkyl group can be obtained by alkylating 4-methylpyrazoles wherein R is a hydrogen atom according to the known method

According to the present invention, 4-methylpyrazoles of the formula (II) can be obtained easily at high yield from inexpensive 2,3-dichloro-2-methylpropanal and the hydrazines of the formula (I).

Particularly, the present invention is effective as the process for preparing 1,4-dimethylpyrazole which is useful as the intermediate for herbicides for use in corn field (Japanese Provisional Patent Publication No. 208977/1985).

The present invention is described in detail below by referring to Examples.

### Reference example 1

Under shielding of light, into a mixed solution of 75.3 g (1 mole) of 93 % methacrolein and 75.3 g of 1,2-dichloroethane, 71 g of chlorine gas was blown under stirring for 3 hours, while maintaining the reaction temperature at 10 °C or lower.

After completion of the reaction, the mixture was stirred at 0 - 5 °C for one hour and further nitrogen gas was blown for 10 minutes to expel the dissolved chlorine gas in the reaction mixture to give 217.7 g of the reaction mixture containing 61.5 % by weight of 2,3-dichloro-2-methylpropanal. Yield: 95 %.

### Example 1

Into a mixture of 34.5 g (0.263 mole) of an aqueous 35 % by weight methylhydrazine and 35 g of 1,2-dichloroethane, a mixed solution of 54.4 g of the reaction mixture containing 2,3-dichloro-2-methylpropanal obtained in Reference example 1 and 35 g of 1,2-dichloroethane was added dropwise under stirring for 2 hours, while maintaining the reaction temperature at 40 to 48 °C.

After the dropwise addition, the mixture was further stirred at 45 to 48°C for 3 hours, at 50 °C for 2 hours, and then returned to room temperature.

After completion of the reaction, 148.1 g of a 25.7 % aqueous potassium carbonate solution was added to the reaction mixture.

After separation of an organic layer, an aqueous layer was extracted twice with 20 g of 1,2-dichloroethane. The extracts and the above organic layer were combined, washed with 10 g of water and dried to obtain an extract.

The extract was analyzed by gas chromatography to find that 17.5 g of 1,4-dimethylpyrazole was contained therein.

The yield based on methacrolein was 73 %. The yield based on 2,3-dichloro-2-methylpropanal was 76.8 %.

After evaporation of the solvent from the extract, distillation was carried out to give 17.0 g of 1,4-dimethylpyrazole of 98.3 % purity at a boiling point in the range of 146.5 to 149 °C.

### Example 2

After evaporation of 1,2-dichloroethane from 21.8 g of the reaction mixture containing 2,3-dichloro-2-methylpropanal obtained in Reference example 1, 40 g of an aqueous 35 % by weight hydrochloric acid solution was added.

Into the mixture was added dropwise 13.8 g (0.105 mole) of a 35 % by weight aqueous methylhydrazine for 2 hours under stirring, while maintaining the reaction temperature at room temperature.

After the dropwise addition, stirring was further continued at 80 to 82°C for 4.5 hours and then returned to room temperature.

After completion of the reaction, the treatment was carried out in the same manner as in Example 1, followed by analysis by gas chromatography. As the result, 6.3 g of 1,4-dimethylpyrazole was found to be contained.

The yield based on methacrolein was 65.6 %. The yield base on 2,3-dichloro-2-methylpropanal was 69.0 %.

### Example 3

Into a mixture of 9.4 g (0.2 mole) of 98 % by weight methylhydrazine and 10 g of diethyl ether was added 14.1 g (purity 92.9 %, 0.093 mole) of 2,3-dichloro-2-methylpropanal under stirring for 2 hours, while maintaining the reaction temperature at room temperature.

After completion of the dropwise addition, the mixture was further stirred at 40 °C for 2 hours and then returned to room temperature.

After completion of the reaction, the treatment was carried out in the same manner as in Example 1 except for using diethyl ether as the extraction solvent of the aqueous layer, followed by analysis by gas chromatography. As the result, 7.4 g of 1,4-dimethylpyrazole was found to be contained.

The yield based on 2,3-dichloro-2-methylpropanal was 83 %.

### Example 4

Into a mixture of 38.9 g (0.525 mole) of isopropylhydrazine, 70 g of 1,2-dichloroethane and 60 g of water was added dropwise a mixed solution of 107.1 g (0.5 mole) of a 1,2-dichloroethane solution containing 65.8 % by weight of 2,3-dichloro-2-methylpropanal and 70 g of 1,2-dichloroethane under stirring for 3 hours, while maintaining the reaction temperature at 40 to 45 °C.

After completion of the dropwise addition, the mixture was further stirred at 50 to 55 °C for 4 hours, at 70 to 80 °C for one hour and thereafter returned to room temperature.

After completion of the reaction, the treatment was carried out in the same manner as in Example 1, the solvent was evaporated from the extract and distillation was carried out to give 32.8 g of 1-isopropyl-4-methylpyrazole at a boiling point range of 61 - 65 °C/18 mmHg.

The yield based on 2,3-dichloro-2-methylpropanal was 53 %.

### Example 5

Into 10.5 g (0.21 mole) of hydrazine monohydrate was added dropwise 28.6 g (purity 98.7 %, 0.2 mole) of 2,3-dichloro-2-methylpropanal under stirring for 4 hours, while maintaining the reaction temperature at 78 to 80 °C.

After completion of the dropwise addition, the mixture was further stirred at 78 to 80 °C for 2 hours, refluxed at 120 to 125 °C and then returned to room temperature.

After completion of the reaction, the treatment was carried out in the same manner as in Example 1 except for carrying out extraction of the aqueous layer with 20 g of chloroform as the extraction solvent for 3 times, followed by analysis by gas chromatography. As the result, 11.7 g of 4-methylpyrazole was found to be contained.

The yield based on 2,3-dichloro-2-methylpropanal was 71.3 %.

### Example 6

Into a mixture of 3.0 g (0.06 mole) of hydrazine monohydrate and 20 g of o-dichlorobenzene was added dropwise 4.4 g of 2,3-dichloro-2-methylpropanal (purity 96.1 %, 0.03 mole) under stirring for 1.5 hours, while maintaining the reaction temperature at 100 °C.

After completion of the dropwise addition, the mixture was further stirred at 100 °C for 1.5 hours, refluxed at 143 to 160 °C for 4.5 hours and then returned to room temperature.

After completion of the reaction, the treatment was carried out in the same manner as in Example 1 except for carrying out extraction of the aqueous layer with 20 g of chloroform as the extraction solvent for 3 times, followed by analysis by liquid chromatography. As the result, 1.7 g of 4-methylpyrazole was found to be contained.

The yield based on 2,3-dichloro-2-methylpropanal was 69 %.

### Example 7

Into a mixture of 4.4 g (purity 96.1 %, 0.03 mole) of 2,3-dichloro-2-methylpropanal and 15 g of acetic acid was added dropwise 2.25 g (0.045 mole) of hydrazine monohydrate under stirring for 10 minutes, while maintaining the reaction temperature at 30 °C or lower.

After completion of the dropwise addition, the mixture was further stirred at 30 °C or lower for 5 hours, refluxed at 118 to 120 °C for 2.5 hours and then returned to room temperature.

After completion of the reaction, the treatment was carried out in the same manner as in Example 1 except for carrying out extraction of the aqueous layer with 20 g of chloroform as the extraction solvent for 3 times, followed by analysis by liquid chromatography. As the result, 1.2 g of 4-methylpyrazole was found to be contained.

The yield based on 2,3-dichloro-2-methylpropanal was 49 %.

### Example 8

Into a mixture of 22.9 g (0.212 mole) of phenylhydrazine, 28 g of 1,2-dichloroethane and 18 g of water was added dropwise a mixed solution of 30 g (purity 94.7 %, 0.202 mole) of 2,3-dichloro-2-methylpropanal and 43 g of 1,2-dichloroethane under stirring for 2 hours, while maintaining the reaction temperature at 40 to 48 °C.

After completion of the dropwise addition, the mixture was further stirred at 45 to 48 °C for 1.5 hours, at 50 °C for one hour and then returned to room temperature.

After completion of the reaction, 118.6 g of a 25.7 % aqueous potassium carbonate solution was added into the reaction mixture.

After separation of an organic layer, an aqueous layer was extracted twice with 20 g of 1,2-dichloroethane. The extract and the above organic layer were combined and dried to obtain an extract.

Analysis of the extract by gas chromatography gave the result that 16.6 g of 1-phenyl-4-methylpyrazole was found to be contained.

The yield based on 2,3-dichloro-2-methylpropanal was 52 %.

### Example 9

Into a mixture of 34.5 g (0.263 mole) of 35 % by weight methylhydrazine and 100 g of 1,2-dichloroethane was added dropwise 38.6 g (0.25 mole) of 91.3 % by weight 2,3-dichloro-2-methylpropanal under stirring for one hour, while maintaining the reaction temperature at 50 to 55 °C. At the same time, pH of the mixture was adjusted to 7 to 8 by adding dropwise a 40 % aqueous sodium hydroxide solution. After completion of the dropwise addition, the mixture was further stirred at 50 to 55 °C and a pH of 7 to 8 for 7.5 hours, at 60 °C and a pH of 7 to 8 for 8 hours and then returned to room temperature. Precipitated sodium chloride was removed by filtration, and an organic layer was separated, an aqueous layer was extracted twice with 10 g of 1,2-dichloroethane. The extract and the above organic layer were combined and analyzed by gas chromatography. As the result, 18.3 g of 1,4-dimethylpyrazole was found to be contained. The yield based on 2,3-dichloro-2-methylpropanal was 76 %.

### Example 10

Into a mixture of 12.32 g (0.26 mole) of 98 % by weight methylhydrazine, 55.55 g (0.55 mole) of triethylamine and 120 g of 1,2-dichloroethane was added dropwise 38.61 g (0.25 mole) of 91.3 % by weight 2,3-dichloro-2-methylpropanal under stirring for one hour, while maintaining the reaction temperature at 40 °C. During the dropwise addition, exothermic heat was observed and the temperature of the reaction mixture raised to 87 °C at the maximum. After completion of the dropwise addition, the mixture was further stirred at 40 °C for 2 hours, at 50 °C for 3 hours and then returned to room temperature. Precipitated triethylamine hydrochloride was removed by filtration, and the filtrate was analyzed by gas chromatography to give the result that 21.1 g of 1,4-dimethylpyrazole was found to be contained. The yield based on 2,3-dichloro-2-methylpropanal was 88 %.

### Example 11

Into a mixture of 34.5 g (0.263 mole) of 35 % by weight methylhydrazine, 2.53 g (0.025 mole) of triethylamine and 75 g of 1,2-dichloroethane was added dropwise 62.28 g (0.25 mole) of 1,2-dichloroethane solution containing 56.6 % by weight of 2,3-dichloro-2-methylpropanal under stirring for 3 hours, while maintaining the reaction temperature at 50 to 55 °C. At the same time, pH of the mixture was adjusted to 7 to 8 by adding dropwise a 40 % aqueous sodium hydroxide solution.

After completion of the dropwise addition, the mixture was further stirred at 50 °C and a pH of 7 to 8 for 3 hours. Subsequently, the reaction mixture was acidified to pH 4 to 5 with 35 % hydrochloric acid and stirred for 3 hours at 50 °C. Finally, the pH of the reaction mixture was controlled to 8 to 9 by using NaOH solution and stirred for 3 hours at 50 °C and then returned to room temperature. To the reaction mixture were added 30 g of water and 15 g of 1,2-dichloroethane and after an organic layer was separated, an aqueous layer was washed twice with 10 g of 1,2-dichloroethane. The extract and the above organic layer were combined and analyzed by gas chromatography. As the result, 20.4 g of 1,4-dimethylpyrazole was found to be contained. The yield based on 2,3-dichloro-2-methylpropanal was 85 %.

### Example 12

Into a mixture of 2.5 g (0.053 mole) of 98 % by weight methylhydrazine, 10 g (0.099 mole) of triethylamine and 50 g of methanol was added dropwise 7 g (0.05 mole) of 2,3-dichloro-2-methylpropanal under stirring for one hour, while maintaining the reaction temperature at 20 °C. After completion of the dropwise addition, the mixture was further stirred at 40 °C for 2 hours and then returned to room temperature. After allowed to stand overnight, and after addition of acetonitrile, the mixture was analyzed by liquid chromatography to give the result that 4.5 g of 1,4-dimethylpyrazole was found to be contained. The yield based on 2,3-dichloro-2-methylpropanal was 94 %.

### Example 13

Into a mixture of 34.5 g (0.263 mole) of 35 % by weight aqueous methylhydrazine solution, 3.03 g (0.025 mole) of 5-ethyl-2-methylpyridine and 75 g of 1,2-dichloroethane was added dropwise 38.3 g (0.25 mole) of 92.0 % by weight 2,3-dichloro-2-methylpropanal under stirring for 3 hours, while maintaining the reaction temperature at 50 to 55 °C. At the same time, pH of the mixture was adjusted to 7 to 8 by adding dropwise a 40 % aqueous sodium hydroxide solution. After completion of the dropwise addition, the mixture was further stirred at 50 °C and a pH of 7 to 8 for 3 hours. Subsequently, the reaction mixture was acidified to pH 4 to 5 with 35 % hydrochloric acid and stirred for 3 hours at 50 °C. Finally, the pH of the reaction mixture was controlled to 8 to 9 by using NaOH solution and stirred for 2 hours at 50 °C and then returned to room temperature. To the reaction mixture were added 30 g of water and 50 g of 1,2-dichloroethane and treated in the same manner as in Example 10. The reaction mixture was analyzed by gas chromatography to give the result that 19.7 g of 1,4-dimethylpyrazole was found to be contained. The yield based on 2,3-dichloro-2-methylpropanal was 82 %.

### Example 14

Into a mixture of 34.5 g (0.263 mole) of 35 % by weight aqueous methylhydrazine solution, 4.0 g (0.013 mole) of tetrabutylammonium bromide and 100 g of 1,2-dichloroethane was added dropwise 72.5 g (0.25 mole) of 1,2-dichloroethane solution containing 48.6 % by weight 2,3-dichloro-2-methylpropanal under stirring for 2 hours, while maintaining the reaction temperature at 50 to 55 °C. At the same time, pH of the mixture was adjusted to 7 to 8 by adding dropwise a 40 % aqueous sodium hydroxide solution. After completion of the dropwise addition, the mixture was further stirred at 50 °C and a pH of 7 to 8 for 3 hours. Subsequently, the reaction mixture was acidified to pH 4 to 5 with 35 % hydrochloric acid and stirred for 2 hours at 50 °C. Finally, the pH of the reaction mixture was controlled to 8 to 9 by using NaOH solution and stirred for 6 hours at 50 °C and then returned to room temperature. To the reaction mixture were added 30 g of water and 50 g of 1,2-dichloroethane and treated in the same manner as in Example 10. The reaction mixture was analyzed by gas chromatography to give the result that 19.8 g of 1,4-dimethylpyrazole was found to be contained. The yield based on 2,3-dichloro-2-methylpropanal was 82 %.

### Comparative example 1

Into 34.5 g (0.263 mole) of a 35 % by weight aqueous methylhydrazine solution were added dropwise 35.7 g of 2,3-dichloro-2-methylpropanal and 44.4 g of a 45 % by weight aqueous sodium hydroxide solution at the same time under stirring for one hour, while maintaining the reaction temperature at 27 to 32 °C.

After completion of the dropwise addition, the mixture was further stirred at 27 to 32 °C for 2 hours, at 65 to 70 °C for one hour, and then returned to room temperature.

After filtration of the solids, the filtrate was extracted once with 100 ml of 1,2-dichloroethane and twice with 20 ml of the same.

The extract was washed with 10 g of water, and then dried to obtain an extract.

Analysis of the extract gave the result that 0.6 g of 1,4-dimethylpyrazole was contained. The yield based on methacrolein was 2.5 %.

## Claims

1. A process for preparing 4-methylpyrazoles represented by the formula (II): 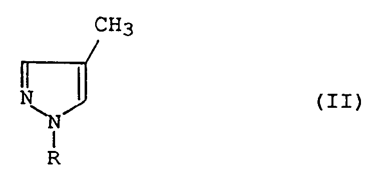 wherein R represents hydrogen, alkyl having 1 to 4 carbon atoms, phenyl, 2-methylphenyl, 3-methylphenyl, 4-methylphenyl, 2-ethylphenyl, 3-ethylphenyl, 4-ethylphenyl, 2-n-propylphenyl, 2-i-propylphenyl, 3-n-propylphenyl, 3-i-propylphenyl, 4-n-propylphenyl, 4-i-propylphenyl, 2-t-butyl-phenyl, 4-n-butylphenyl, 4-i-butylphenyl, 4-t-butylphenyl, 2,4-dimethylphenyl, 2,6-dimethylphenyl, 2,4-diethylphenyl, 2,6-diethylphenyl, 2-methoxyphenyl, 3-methoxyphenyl, 4-methoxyphenyl, 2-ethoxyphenyl, 3-ethoxyphenyl, 4-ethoxyphenyl, 2-n-propoxyphenyl, 2-i-propoxyphenyl, 3-n-propoxyphenyl,4-n-propoxyphenyl, 4-i-propoxyphenyl, 2-n-butoxyphenyl, 4-n-butoxyphenyl, 4-i-butoxyphenyl, 4-t-butoxyphenyl, 2-chlorophenyl, 3-chlorophenyl, 4-chlorophenyl, 2-fluorophenyl, 3-fluorophenyl, 4-fluorophenyl, 2,4-dichlorophenyl, 2,4-difluorophenyl, 2,6-dichlorophenyl, 2,6-difluorophenyl, 2-nitrophenyl, 3-nitrophenyl, 4-nitrophenyl, 2-difluoromethoxyphenyl, 3-difluoromethoxyphenyl, 4-difluoromethoxyphenyl, 2-pyridyl, 3-methyl-2-pyridyl, 4-methyl-2-pyridyl, 5-methyl-2-pyridyl, 6-methyl-2-pyridyl, 3-chloro-2-pyridyl, 4-chloro-2-pyridyl, 5-chloro-2-pyridyl, 6-chloro-2-pyridyl, 3,5-dichloro-2-pyridyl, 3-trifluoromethyl-2-pyridyl, 5-trifluoromethyl-2-pyridyl, 3-pyridyl, 2-methyl-3-pyridyl, 4-methyl-3-pyridyl, 5-methyl-3-pyridyl, 6-methyl-3-pyridyl, 2-chloro-3-pyridyl, 5-chloro-3-pyridyl, 6-chloro-3-pyridyl, 4-pyridyl, 2-methyl-4-pyridyl, 3-methyl-4-pyridyl, 2-chloro-4-pyridyl, or 3-chloro-4-pyridyl which comprises reacting 2,3-dichloro-2-methyl-propanal with hydrazines represented by the formula (I):
RNHNH₂ (I)
(wherein R is the same as defined above), the reaction being carried out at a temperature of -10 to 160°C, in the presence or absence of a solvent, with the pH being controlled at a value of 11 or lower when reaction is carried out in water or a solvent containing water, or in a two layer system using water and a water-insoluble organic solvent.

2. A process according to Claim 1, wherein R is a methyl group.

3. A process according to Claim 1, wherein R is a hydrogen atom.

4. A process according to Claim 1, wherein an inorganic or organic dehydrochlorinating agent is used.

5. A process according to any one of Claims 1 to 4, wherein the reaction is carried out in the presence of at least one solvent selected from the group consisting of hexane, heptane, methylene chloride, chloroform, carbon tetrachloride, dichloroethane, tetrachloroethane, benzene, toluene, xylene, chlorobenzene, dichlorobenzene, methanol, ethanol, diethyl ether, tetrahydrofuran, dioxane, acetic acid, propionic acid, butyric acid, water, aqueous hydrochloric acid solution and aqueous sulfuric acid solution.

6. A process according to Claim 5, wherein the solvent is at least one selected from the group consisting of water, aqueous hydrochloric acid solution, acetic acid, dichloroethane, o-dichlorobenzene and diethyl ether.

7. A process according to any one of Claims 1 to 6 wherein the reaction is carried out by controlling the pH of the reaction mixture to 4 to 9.

8. A process according to any one of Claims 1 to 7 wherein an organic base is added as a catalyst.

9. A process according to Claim 8 wherein the catalyst is one or more kinds selected from trimethylamine, triethylamine, tri-n-propylamine, tributylamine, tri-n-octylamine, diisopropylethylamine, dimethylcyclohexylamine, diethylcyclohexylamine, N,N-dimethylaniline, N,N-diethylaniline, pyridine, picoline and ethylmethylpyridine.

10. A process according to Claim 1 wherein reaction is carried out in a two layer system using water and a water-insoluble organic solvent, and a phase-transfer catalyst is added.

11. A process according to Claim 10, wherein the phase-transfer catalyst is one or more kinds selected from tetrabutylammonium bromide, triethylbenzylammonium chloride, tetrabutylammonium hydrosulfate, tetraoctylmethylammonium chloride, cetyltrimethylammonium bromide, tetrabutylphosphonium chloride, tetraphenylphosphonium chloride, 18-crown-6 and PEG-1000.

## Patentansprüche

1. Verfahren zum Herrichten eines 4-Methylpyrazoles, dargestellt durch die Formel (II): 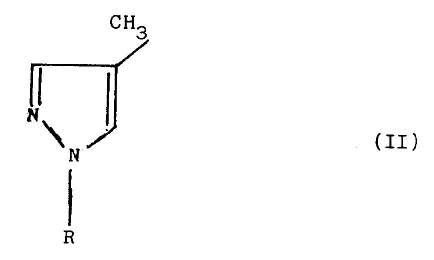 wobei R darstellt: Wasserstoff, Alkyl mit 1 bis 4 Kohlenstoffatomen, Phenyl, 2-Methylphenyl, 3-Methylphenyl, 4-Methylphenyl, 2-Ethylphenyl, 3-Etylphenyl, 4-Ethylphenyl, 2-n-Propylphenyl, 2-i-Propylphenyl, 3-n-Propylphenyl, 3-i-Propylphenyl, 4-n-Propylphenyl, 4-i-Propylphenyl, 2-t-Butylphenyl, 4-n-Butylphenyl, 4-i-Butylphenyl, 4-t-Butylphenyl, 2,4-Dimethylphenyl, 2, 6-Dimethylphenyl, 2,4- Diethylphenyl, 2, 6-Diethylphenyl, 2-Methoxyphenyl, 3-Methoxyphenyl, 4-Methoxyphenyl, 2-Ethoxyphenyl, 3-Ethoxyphenyl, 4-Ethoxyphenyl, 2-n-Propoxyphenyl, 2-i-Propoxyphenyl, 3-n-Propoxyphenyl, 4-n-Propoxyphenyl, 4-i-Propoxyphenyl, 2-n-Butoxyphenyl, 4-n-Butoxyphenyl, 4-i-Butoxyphenyl, 4-t-Butoxyphenyl, 2-Chlorphenyl, 3-Chlorphenyl, 4-Chlorphenyl, 2-Fluorphenyl, 3-Fluorphenyl, 4-Fluorphenyl, 2,4- Dichlorphenyl, 2,4-Difluorphenyl, 2,6-Dichlorphenyl, 2,6-Difluorphenyl, 2-Nitrophenyl, 3-Nitrophenyl, 4-Nitrophenyl, 2-Difluormethoxyphenyl, 3-Difluormethoxyphenyl, 4-Difluormethoxyphenyl, 2-Pyridyl, 3-Methyl-2-Phyridyl, 4-Methyl-2-Pyridyl, 5-Methyl-2-Pyridyl, 6-Methyl-2-Pyridyl, 3-Chlor-2-Pyridyl, 4-Chlor-2-Pyridyl, 5-Chlor-2-Pyridyl, 6-Chlor-2-Pyridyl, 3, 5-Dichlor-2-Pyridyl, 3-Trifluormethyl-2-Pyridyl, 5-Trifluormethyl-2-Pyridyl, 3-Pyridyl, 2-Methyl-3-Pyridyl, 4-Methyl-3-Pyridyl, 5-Methyl-3-Pyridyl, 6-Methyl-3-Pyridyl, 2-Chlor-3-Pyridyl, 5-Chlor-3-Pyridyl, 6-Chlor-3-Pyridyl, 4-Pyridyl, 2-Methyl -4-Pyridyl, 3-Methyl-4-Pyridyl, 2-Chlor-4-Pyridyl, oder 3-Chlor-4-Pyridyl, das reaktionsfähiges 2, 3-Dichlor-2-Methyl-Propanal bzw. -propylaldehyd mit Hydrazinen oder Diamiden enthält, welche durch die Formel (I):
RNHNH₂ (I)
dargestellt ist (wobei R mit der vorstehenden Definition übereinstimmt), wobei die Reaktion bei einer Temperatur zwischen - 10 und + 160° C mit oder ohne Lösungsmittel durchgeführt wird, wobei der pH-Wert auf einen Wert von 11 oder niedriger gesteuert wird, wenn die Reaktion in Wasser oder in einem Wasser-enthaltenden Lösungsmittel oder in einem Zwei-Schichten-System unter Verwendung von Wasser und einem in Wasser nicht löslichen organischen Lösungsmittel durchgeführt wird.

2. Verfahren nach Anspruch 1, wobei R eine Methyl-Gruppe ist.

3. Verfahren nach Anspruch 1, wobei R ein Wasserstoffatom ist.

4. Verfahren nach Anspruch 1, wobei ein anorganisches oder ein organisches Dehydrochloriermittel eingesetzt wird.

5. Verfahren nach einem der Ansprüche 1 bis 4, wobei die Reaktion in Anwesenheit wenigstens eines Lösungsmittels durchgeführt wird, das aus der Gruppe ausgewählt wird, welche Hexan, Heptan, Methylenchlorid, Chloroform, Kohlenstofftetrachlorid, Dichlorethan, Tetrachlorethan, Benzol, Toluol, Xylol, Chlorbenzol, Dichlorbenzol, Methanol, Ethanol, Diethylether, Tetrahydropuran, Dioxan, Essigsäure, Propionsäure, Buttersäure, Wasser, wässrige Salzsäurelösung und wässrige Schwefelsäurelösung umfaßt.

6. Verfahren nach Anspruch 5, dadurch gekennzeichnet, daß als Lösungsmittel wenigstens eines aus der Gruppe ausgewählt wird, die Wasser, wässrige Salzsäurelösung, Essigsäure, Dichlorethan, O-Dichlorbenzol und Diethylether umfaßt.

7. Verfahren nach einem der Ansprüche 1 bis 6, dadurch gekennzeichnet, da die Reaktion durch das Steuern bzw. unter Steuerung des pH-Wertes der Reaktionsmischung zwischen 4 und 9 durchgeführt wird.

8. Verfahren nach einem der Ansprüche 1 bis 7, dadurch gekennzeichnet, da eine organische Base als Katalysator beigefügt wird.

9. Verfahren nach Anspruch 8, dadurch gekennzeichnet, daß der Katalysator aus einer oder mehreren Substanzen besteht, die aus Trimethylamin, Triethylamin, Tri-n-Propylamin, Tributylamin, Tri-n-Octylamin, Diisopropylethylamin, Dimethylcyclohexylamin, N,N-Dimethylanilin, N,N-Diethylanilin, Pyridin, Picolin, oder Methylpyridin und Ethylmethylpyridin gewählt ist.

10. Verfahren nach Anspruch 1, dadurch gekennzeichnet, daß die Reaktion in einem Zwei-Schichten-System unter Verwendung von Wasser und einem Wasser-unlöslichen organischen Lösungsmittel ausgeführt wird, und da ein Phasen-Übergangs-Katalysator zugesetzt wird.

11. Verfahren nach Anspruch 10, dadurch gekennzeichnet, daß der Phasenübergangs-Katalysator aus einer oder aus mehreren Substanzen besteht, die aus Tetrabutylammoniumbromid, Triethylbenylammoniumchlorid, Tetrabutylammoniumhydrosulfat, Tetraoctylmethylammoniumchlorid, Cetyltrimethylammoniumbromid, Tetrabutylphosphoniumchlorid, Tetraphenylphosphoniumchlorid, 18-Krone-6 oder 18-crown-6 sowie PEG-1000 ausgewählt werden.

## Revendications

1. Procédé de préparation de 4-méthylpyrazoles représentés par la formule (II) : 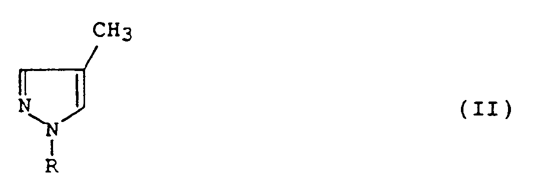 où R représente de l'hydrogène, un groupe alkyle contenant 1 à 4 atomes de carbone, du phényle, 2-méthylphényle, 3-méthylphényle, 4-méthylphényle, 2-éthylphényle, 3-éthylphényle, 4-éthylphényle, 2-n-propylphényle, 2-i-propylphényle, 3-n-propylphényle, 3-i-propylphényle, 4-n-propylphényle, 4-i-propylphényle, 2-t-butylphényle, 4-n-butylphényle, 4-i-butylphényle, 4-t-butylphényle, 2,4-diméthylphényle, 2,6-diméthylphényle, 2,4-diéthylphényle, 2,6-diéthylphényle, 2-méthoxyphényle, 3-méthoxyphényle, 4-méthoxyphényle, 2-éthoxyphényle, 3-éthoxyphényle, 4-éthoxyphényle, 2-n-propoxyphényle, 2-i-propoxyphényle, 3-n-propoxyphényle, 4-n-propoxyphényle, 4-i-propoxyphényle, 2-n-butoxyphényle, 4-n-butoxyphényle, 4-i-butoxyphényle, 4-t-butoxyphényle, 2-chlorophényle, 3-chlorophényle, 4-chlorophényle, 2-fluorophényle, 3-fluorophényle, 4-fluorophényle, 2,4-dichlorophényle, 2,4-difluorophényle, 2,6-dichlorophényle, 2,6-difluorophényle, 2-nitrophényle, 3-nitrophényle, 4-nitrophényle, 2-difluorométhoxyphényle, 3-difluorométhoxyphényle, 4-difluorométhoxyphényle, 2-pyridyle, 3-méthyl-2-pyridyle, 4-méthyle-2-pyridyle, 5-méthyl-2-pyridyle, 6-méthyl-2-pyridyle, 3-chloro-2-pyridyle, 4-chloro-2-pyridyle, 5-chloro-2-pyridyle, 6-chloro-2-pyridyle, 3,5-dichloro-2-pyridyle, 3-trifluorométhyl-2-pyridyle, 5-trifluorométhyl-2-pyridyle, 3-pyridyle, 2-méthyl-3-pyridyle, 4-méthyl-3-pyridyle, 5-méthyl-3-pyridyle, 6-méthyl-3-pyridyle, 2-chloro-3-pyridyle, 5-chloro-3-pyridyle, 6-chloro-3-pyridyle, 4-pyridyle, 2-méthyl-4-pyridyle, 3-méthyl-4-pyridyle, 2-chloro-4-pyridyle, ou du 3-chloro-4-pyridyle qui comprend la réaction de 2,3-dichloro-2-méthyl-propanal avec des hydrazines représentées par la formule (I) :
RNHNH₂ (I)
(où R est identique à la définition ci-dessus), la réaction se déroulant à une température comprise entre -10 °C et 160 °C, en présence ou en l'absence d'un solvant, le pH étant régulé à une valeur de 11 ou moins lorsque la réaction se déroule dans l'eau ou un solvant contenant de l'eau, ou dans un système à deux couches utilisant de l'eau et un solvant organique insoluble dans l'eau.

2. Procédé selon la revendication 1, dans lequel R est un groupe méthyle.

3. Procédé selon la revendication 1, dans lequel R est un atome d'hydrogène.

4. Procédé selon la revendication 1, dans lequel un agent déchlorhydratant organique ou inorganique est utilisé.

5. Procédé selon l'une quelconque des revendications 1 à 4, dans lequel la réaction se déroule en présence d'au moins un solvant choisi dans le groupe consistant en hexane, heptane, chlorure de méthylène, chloroforme, tétrachlorure de carbone, dichloroéthane, tétrachloroéthane, benzène, toluène, xylène, chlorobenzène, dichlorobenzène, méthanol, éthanol, éther diéthylique, tétrahydrofuranne, dioxane, acide acétique, acide propionique, acide butyrique, eau, solution aqueuse d'acide chlorhydrique et solution aqueuse d'acide sulfurique.

6. Procédé selon la revendication 5, dans lequel le solvant est au moins un solvant choisi dans le groupe consistant en eau, solution aqueuse d'acide chlorhydrique, acide acétique, dichloroéthane, o-dichlorobenzène et éther diéthylique.

7. Procédé selon l'une quelconque des revendications 1 à 6, dans lequel la réaction se déroule en régulant le pH du mélange réactionnel entre 4 et 9.

8. Procédé selon l'une quelconque des revendications 1 à 7, dans lequel une base organique est ajoutée comme catalyseur.

9. Procédé selon la revendication 8 dans lequel le catalyseur est une ou plusieurs espèces choisies entre la triméthylamine, triéthylamine, tri-n-propylamine, tributylamine, tri-n-octylamine, diisopropyléthylamine, diméthylcyclohexylamine, diéthylcyclohexylamine, N,N-diméthylaniline, N,N-diéthylaniline, pyridine, picoline et éthylméthylpyridine.

10. Procédé selon la revendication 1 dans lequel la réaction se déroule dans un système à deux couches utilisant de l'eau et un solvant organique Insoluble dans l'eau, et un catalyseur de transfert de phase est ajouté.

11. Procédé selon la revendication 10, dans lequel le catalyseur de transfert de phase est une ou plusieurs espèces choisies parmi le bromure de tétrabutylammonium, le chlorure de triéthylbenzylammonium, l'hydrosulfate de tétrabutylammonium, le chlorure de tétraoctylméthylammonium, le bromure de cétyltriméthylammonium, le chlorure de tétrabutylphosphonium, le chlorure de tétraphénylphosphonium, le 18-couronne-6 et le PEG-1000.
